# FASCICULE DE BREVET EUROPEEN

(11) **EP 2 542 532 B1**
(45) Date de publication et mention de la délivrance du brevet: **03.01.2018**
(21) Numéro de dépôt: 11700249.3
(22) Date de dépôt: 07.01.2011
(51) Int. Cl.: C07D 233/58, C07F 15/00, C08G 77/08

(54) **PROCÉDÉ DE PRÉPARATION DE CARBÈNE EN SOLUTION ET FORME STABLE DE CARBÈNE OBTENU PAR CE PROCÉDÉ**
VERFAHREN ZUR HERSTELLUNG VON CARBEN IN EINER LÖSUNG UND STABILE FORM VOM DURCH DIESES VERFAHREN HERGESTELLTEN CARBEN
METHOD FOR PREPARING CARBENE IN SOLUTION AND STABLE FORM OF CARBENE OBTAINED BY SAID METHOD

(30) Priorité: 08.01.2010 FR 1050108
(43) Date de publication de la demande: 09.01.2013
(73) Titulaire: Bluestar Silicones France SAS, 69003 Lyon (FR); CNRS, 75016 Paris (FR)
(72) Inventeur: MALIVERNEY, Christian, F-69690 Saint Julien Sur Bibost (FR); SAINT-JALMES, Laurent, F-69390 Vourles (FR); BACEIREDO, Antoine, J., 31500 Toulouse (FR); KATO, Tsuyoshi, 31400 Toulouse (FR); GOJON, Sophie, 92500 Rueil-Malmaison (FR)
(86) Numéro de dépôt international: PCT/EP2011/050175
(87) Numéro de publication internationale: WO 2011/083146

(56) Documents cités:
- KNISHEVITSKY A V ET AL: "Copper(I) halide complexes of the new 4,4'-bridged heteroaromatic biscarbenes of the 1,2,4-triazole series", JOURNAL OF ORGANOMETALLIC CHEMISTRY, ELSEVIER-SEQUOIA S.A. LAUSANNE, CH, vol. 693, no. 8-9, 15 avril 2008 (2008-04-15), pages 1405-1411, XP022590097, ISSN: 0022-328X, DOI: DOI:10.1016/J.JORGANCHEM.2007.07.056 [extrait le 2008-04-05]
- CHEN HAO ET AL: "Proton affinities of N-heterocyclic carbene super bases.", ORGANIC LETTERS 1 SEP 2005 LNKD- PUBMED:16119939, vol. 7, no. 18, 1 septembre 2005 (2005-09-01), pages 3949-3952, XP002627477, ISSN: 1523-7060
- SCHOLL M ET AL: "SYNTHESIS AND ACTIVITY OF A NEW GENERATION OF RUTHENIUM-BASED OLEFIN METATHESIS CATALYSTS COORDINATED WITH 1,3-DIMESITYL-4,5-DIHYDROIMIDAZ OL-2-YLIDENE LIGANDS", ORGANIC LETTERS, AMERICAN CHEMICAL SOCIETY, US, vol. 1, no. 6, 1 janvier 1999 (1999-01-01) , pages 953-956, XP000984756, ISSN: 1523-7060, DOI: DOI:10.1021/OL990909Q
- TINA M TRNKA ET AL: "Synthesis and Activity of Ruthenium Alkylidene Complexes Coordinated with Phosphine and N-Heterocyclic Carbene Ligands", JOURNAL OF THE AMERICAN CHEMICAL SOCIETY, AMERICAN CHEMICAL SOCIETY, US, vol. 125, no. 9, 1 juillet 2003 (2003-07-01), pages 2546-2558, XP001537178, ISSN: 0002-7863, DOI: DOI:10.1021/JA021146W [extrait le 2003-02-12]
- Nikolai I. Korotkikh ET AL: "Tandem transformations of 1,2,4-triazol-5-ylidenes into 5-amidino-1,2,4-triazoles", ARKIVOC, 1 January 2007 (2007-01-01), pages 156-172, XP055073628, Retrieved from the Internet: URL:http://www.arkat-usa.org/get-file/2327 9/ [retrieved on 2013-07-31]
- Mohammad Movassaghi ET AL: "N-Heterocyclic Carbene-Catalyzed Amidation of Unactivated Esters with Amino Alcohols", Organic Letters, vol. 7, no. 12, 1 June 2005 (2005-06-01), pages 2453-2456, XP055018304, ISSN: 1523-7060, DOI: 10.1021/ol050773y
- Olivier Coulembier ET AL: "Alcohol Adducts of N -Heterocyclic Carbenes: Latent Catalysts for the Thermally-Controlled Living Polymerization of Cyclic Esters", Macromolecules, vol. 39, no. 17, 1 August 2006 (2006-08-01), pages 5617-5628, XP055065316, ISSN: 0024-9297, DOI: 10.1021/ma0611366

## Description

### Domaine de l'invention

Le domaine de l'invention est celui de la synthèse de carbènes et de leurs applications notamment comme catalyseurs ou ligands dans :
- la préparation de PolyOrganoSiloxanes (POS) par polymérisation par ouverture de cycle et/ou par redistribution et/ou polycondensation de POS,
- l'hydrosilylation d'au moins un PolyOrganoSiloxane -A- (POS) porteur d'insaturation(s) éthylènique(s) et/ou acétylènique(s), à l'aide d'au moins un polyorganohydrogénosiloxane -B-, ou d'autres composés éthyléniquement et/ou acétyléniquement insaturés,
- l'hydrogénation,
- la métathèse d'oléfines,
- le couplage croisé C-C et/ou C-N,
- la synthèse asymétrique ou énantiosélective,
- ou la synthèse organique : réactions d'estérification, trans-estérification, condensation de type aldolisation par exemple.

La présente invention concerne un procédé de préparation de carbène par déprotonation d'un sel précurseur à l'aide d'une base forte.

Dans tout le présent exposé, tout singulier désigne indifféremment un singulier ou un pluriel.

### Arrière-plan technologique et problème technique

Les complexes Platine/carbène sont connus comme catalyseur d'hydrosilylation de PolyOrganoSiloxane (POS) à motifs ≡Si-Vinyle, au moyen de POS à motifs ≡Si-H. A titre d'exemple, on peut citer la demande PCT WO-A-02/098971 qui décrit une composition silicone réticulable en élastomère par hydrosilylation, en présence de catalyseurs au platine formé par un complexe C3 ou C4 :

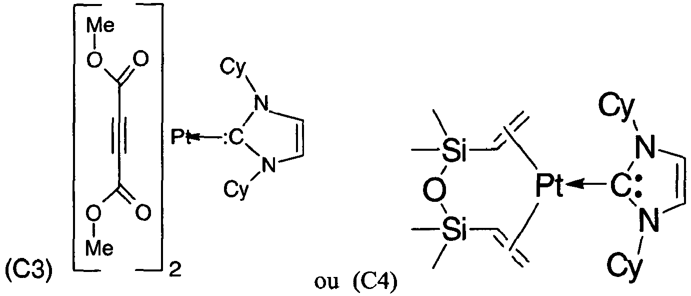

Les carbènes utiles en tant que ligands de métaux catalytiques, sont exploités dans d'autres domaines que celui des silicones. Ainsi, le brevet EP-B-0 971 941 décrit des catalyseurs à base de complexes Ruthénium et Osmium/carbène, pour la métathèse thermique de cyclooléfines.

En outre, un article de J.L. Hedrick et al paru en 2002, (JACS 124, n° 6 p 914-915, 2002), enseigne que des carbènes N-hétérocycliques peuvent être utilisés comme catalyseur de polymérisation d'esters cycliques. Plus précisément, le 1,3-bis-(2,4,6-triméthylphényl)-imidazol-2-ylidène a été testé comme catalyseur de polymérisation du L-lactide, de l'ε caprolactone et du β-butyrolactone, en présence d'un alcool utilisé comme amorceur.

Un article plus récent et plus complet (cf. JACS 125, n° 10 p 3046-3056, 2003) décrit la préparation de carbène in-situ pour éviter les problèmes d'hydrolyse. Les promoteurs utilisés sont de type thiazolium, imidazolium et imidazolinium donnant respectivement des catalyseurs thioazole carbène, imidazole-2-ylidène carbène et imidazolin-2-ylidène carbène, lorsqu'ils sont mis en présence ter-butylate de potassium.

La demande de brevet FR-2864543-A divulgue des carbènes obtenus par déprotonation d'un sel d'imidazolium en présence d'une base forte et utilisés pour la synthèse de silicones polyorganosiloxanes, par polymérisation par ouverture de cycle(s) et/ou redistribution de polyorganosiloxanes linéaires ou cycliques.

La demande de brevet FR-2900153-A divulgue un carbène, le dicyclohexyldiaminocarbène obtenu par déprotonation du triflate d'imidazolium correspondant 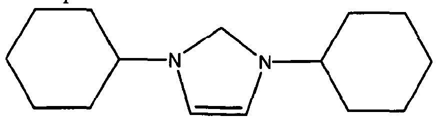 selon le protocole opératoire suivant:

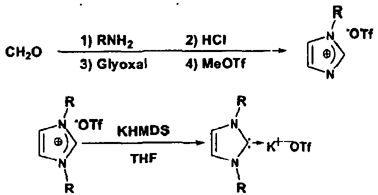

Ce carbène catalyse la polycondensation de silanols du type huile silicone de formule [(CH₃)₂(OH)SiO_{1/2}]-[(CH₃)₂SiO_{2/2}]₁₀-[CH₃)₂(OH)SiO_{1/2}].

Ces carbènes de type NHC obtenus par déprotonation d'un sel d'imidazolium à l'aide d'une base forte dans un solvant de type THF, sont ensuite isolés par filtration afin d'éliminer le sel formé puis recristallisation, voire sublimation.

A cet égard, le brevet EP-1268440-B1 concerne un procédé de préparation de carbènes N-hétérocycliques comportant le chauffage d'un halogénure de dialkylimidazolium avec une base forte sous pression réduite. Le carbène est distillé pendant sa formation avec un appareil de distillation horizontale, éventuellement distillé avec le même appareil une seconde fois. Il peut être isolé pur sous gaz inerte ou dissout dans un solvant inerte (préférentiellement aromatique).

Ce procédé n'est pas industriel car la distillation horizontale qui utilise un appareil de laboratoire n'est possible que pour quelques grammes à quelques dizaines de grammes. De plus, le carbène isolé pur ou en solvant inerte est très sensible à l'oxygène et à la moindre trace d'humidité, et de ce fait a une durée de vie limitée.

Ces carbènes sont des espèces très réactives qui se dégradent à l'air libre, ce qui implique de les conserver et de les manipuler sous atmosphère inerte (argon ou azote). Leur synthèse est elle aussi bien évidemment menée sous atmosphère inerte et le solvant utilisé est préalablement séché et fraîchement distillé.

L'article de O. Coulembier et al.'Macromolecules 2006, 39, 5617-5628 rapporte l'observation d'un mélange de méthanol, d'un carbène de triazole et d'un méthoxytriazole à 90°C.

Par ailleurs, l'article de M.A SCHMIDT et al Tetrahedron letters 49 (2008) 4316-4318 divulgue l'interaction de N,N'-bismesitylimidazolium-2-yl et d'alcools, en présence de benzène :

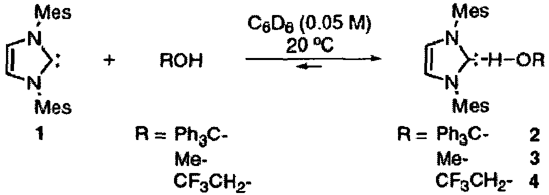

De cette observation, les auteurs déduisent que l'effet stabilisant de ces interactions peut être utilisé pour le stockage de carbènes hétérocycliques azotés, puisque l'élimination des volatils libère ces carbènes hétérocycliques azotés. Dans le cas où l'alcool est le méthanol, cette évaporation des volatils doit s'accompagner d'une désolvatation pour retrouver le complexe carbène-alcool.

Eu égard à cet état de la technique, l'invention vise à satisfaire au moins l'un des objectifs essentiels suivants :
∘ perfectionner la synthèse de carbènes, c'est-à-dire la simplifier, la rendre plus économique et aboutir à une forme liquide ou solide, stable et pure qui constitue un système catalytique qui soit facile à stocker et à utiliser et qui soit aussi plus efficace, plus rentable et plus sélectif que les catalyseurs au carbène de l'art antérieur ;
∘ obtenir des carbènes en solution, stables à l'air libre pendant au moins plusieurs mois ;
∘ fournir de nouveaux complexes métalliques de carbène stables, économiques et performants sur le plan catalytique ;
∘ fournir des procédés et des catalyseurs stables, économiques et performants de :
   a. préparation de PolyOrganoSiloxanes (POS) par polymérisation par ouverture de cycle et/ou par redistribution-polycondensation de POS
   b. hydrosilylation d'au moins un PolyOrganoSiloxane -A- (POS) porteur d'insaturation(s) éthylènique(s) et/ou acétylènique(s), à l'aide d'au moins un polyorganohydrogénosiloxane -B-, ou d'autres composés éthyléniquement et/ou acétyléniquement insaturés
   c. hydrogénation
   d. métathèse d'oléfines
   e. couplage croisé C-C et/ou C-N
   f. synthèse énantiosélective
   g. synthèse organique
∘ fournir des procédés a à g ayant de meilleurs rendements de conversion ;
∘ fournir un système catalytique à base de carbène performant, qui présente au moins l'une des caractéristiques suivantes :
   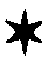 soluble dans les huiles silicones et en particulier les gommes silicones ;
   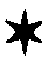 simple et peu coûteux à synthétiser ;
   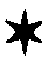 stable ;
   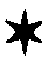 doté d'une bonne tenue à l'hydrolyse ;
   et qui permette :
   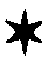 de polymériser des silicones dans des conditions douces (basses températures ≤ 100°C) ;
   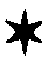 de diminuer les temps de réaction en particulier pour la préparation des huiles visqueuses et des gommes ;
   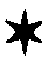 de réduire, voire de supprimer, les résidus de catalyseur et de ses dérivés dans le polymère final, afin de préparer des polymères silicone de forte viscosité et de tenue thermique améliorée, et ce de façon rentable ;
   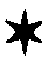 de fonctionnaliser toute une palette de POS ;
   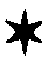 d'améliorer la polydispersité des polymères formés et de privilégier la formation de structures linéaires par rapport aux oligomères cycliques ;
   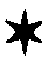 une élimination aisée des éventuels résidus de catalyseurs ;
   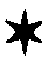 de privilégier la formation de polymères silicone linéaires par rapport à la formation de cycliques ;
   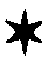 de garantir une haute reproductibilité ;
   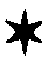 et de limiter la sensibilité à la variabilité des matières premières.

### Brève description de l'invention

Ces objectifs, parmi d'autres, sont atteints par un procédé de préparation d'un catalyseur ou d'un réactif comprenant un carbène et stable à 20° C se présentant sous la forme d'une solution **A** constituée d'au moins un alcool et d'au moins un carbène comprenant les étapes suivantes:
- on fait réagir, de préférence à une température comprise entre 0° C et 100° C, au moins une base forte, éventuellement générée in situ, avec au moins un composé **B** précurseur d'un carbène **C,** ledit composé **B** qui est un sel comprenant une fonction iminium de structure (I) suivante: 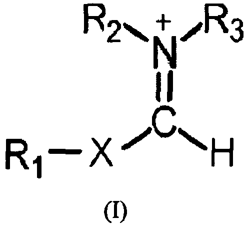 avec :
   - X étant choisi parmi le groupe constitué par : O, S, -N(R) et -P(R), avec R étant un groupement en C₁-C₃₀,
   - R₁, R₂ et R₃ étant des groupements éventuellement substitués, identiques ou différents, en C₁-C₃₀ et comprenant éventuellement un ou plusieurs atomes choisi(s) parmi le groupe constitué par: S, P, Si, N et O,
   - X, R₁, R₂ et R₃ pris deux à deux peuvent former un cycle à 5, 6 ou 7 chainons;
   avec comme conditions suivantes :
   - le choix de la base forte s'effectue de manière à pouvoir effectuer une déprotonation de l'hydrogène de la fonction iminium de structure (I), et
   - la réaction s'effectue dans un solvant qui est un alcool ou un mélange d'alcools. Avantageusement, cette déprotonation s'opère à température ambiante (20°C). Il est du mérite des inventeurs d'avoir osé la synthèse de carbène en milieu alcoolique malgré le préjugé existant sur l'incompatibilité carbène-eau ou alcool. Ceci a permis, de manière tout à fait surprenante et inattendue, de s'affranchir de la lourde contrainte de mise en oeuvre d'un milieu réactionnel anhydre et d'isolement du carbène par sublimation avec de très faibles rendement (<40% massique).

Le procédé selon l'invention est beaucoup plus simple puisque les opérations de purification sont allégées, voire supprimées. Il est également plus économique. Bref, il est beaucoup plus industriel que les procédés connus. Selon l'invention, le composé **B** est choisi parmi les composés de formule générale (III) ou (III') suivante : 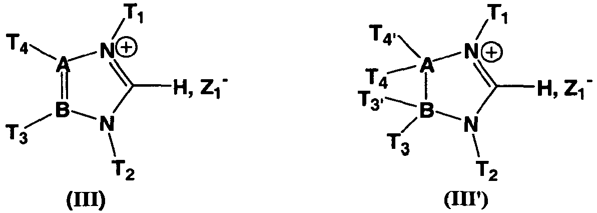 dans laquelle :
- A et B représentent indépendamment C ou N, étant entendu que :
   - dans la formule (III), lorsque A représente N, alors T4 n'est pas présent et lorsque B représente N, alors T3 n'est pas présent ;
   - dans la formule (III'), lorsque A représente N, alors T4 ou T4' n'est pas présent et lorsque B représente N, alors T3 ou T3' n'est pas présent ;
- T3, T3', T4 et T4' représentent indépendamment un atome d'hydrogène; un groupement alkyle ; cycloalkyle éventuellement substitué par un groupe alkyle ou alkoxy ; aryle éventuellement substitué par un groupe alkyle ou alkoxy ; alcényle ; alcynyle ; ou arylalkyle dans lequel la partie aryle est éventuellement substituée par un groupe alkyle ou alkoxy ; ou bien
- T3 et T4 peuvent former ensemble et avec A et B quand ceux-ci représentent chacun un atome de carbone, un aryle étant entendu que dans ce cas T3' et T4' ne sont pas présents ;
- T1 et T2 représentent indépendamment un groupement alkyle ; un groupement alkyle éventuellement substitué par un alkyle ; un groupement alkyle perfluoré ou éventuellement substitué par un groupement perfluoroalkyle ; cycloalkyle éventuellement substitué par un groupe alkyle ou alkoxy; aryle éventuellement substitué par un groupe alkyle ou alkoxy ; alcényle ; alcynyle ; ou arylalkyle dans lequel la partie aryle est éventuellement substituée par un groupe alkyle ou alkoxy ; ou bien
- T1 et T2 représentent indépendamment un radical monovalent de formule (III) suivante :

   -V1-V2 (III)

   dans laquelle :
   - V1 est un groupement divalent hydrocarboné saturé ou non, de préférence un alkylène linéaire ou ramifié en C1-C10, éventuellement substitué,
   - V2 est un groupement monovalent choisi dans le groupe des substituants suivants :
      ◆ alcoxy, -OR^{a} avec R^{a} correspondant à hydrogène, alkyle, aryle ;
      ◆ silyl, -Si(OR^{b})ₓ(R^{c})₃₋ₓ avec R^{b} correspondant à hydrogène, alkyle, silyle ou siloxanyle, R^{c} correspondant à alkyle, aryle et x étant un entier compris entre 0 et 3 ;
      ◆ amine, de préférence -N(R^{a})₂ avec R^{a} correspondant à hydrogène, alkyle, aryle ; ou bien encore ;
- les substituants T1, T2, T3, T3' T4 et T4' peuvent former deux à deux, lorsqu'ils sont situés sur deux sommets adjacents dans les formules (III) et (III'), une chaîne hydrocarbonée saturée ou insaturée.
- Z1 représente indépendamment un anion dérivé d'un acide de Brönsted (acide protique) de préférence choisi dans le groupe comprenant :
   - les acides carboxyliques de formule Gₒ-COOH dans laquelle Gₒ représente un alkyle, et avantageusement un alkyle en C1-C22 ; un aryle, avantageusement un aryle en C6-C18 éventuellement substitué par un ou plusieurs alkyle en C1-C6 ;
   - les acides sulfoniques de formule Gₒ-SO₃H dans laquelle Gₒ est tel que défini ci-dessus ;
   - les acides phosphoriques de formule Gₒ-PO₃H dans laquelle Gₒ est tel que défini ci-dessus ;
   - les acides minéraux suivants : HF, HCl, HBr, HI, H₂SO₄, H₃PO₄, HClO₄ et HBF₄ pris à eux seuls ou en combinaison entre eux ;
   - et leurs mélanges.

Concernant le sel **(III),** l'anion Z₁⁻ est l'anion dérivé d'un acide de Brönsted (acide protique) organique ou minéral. Habituellement, l'anion Z₁⁻ est dérivé d'un acide présentant un pKa inférieur à 6. De préférence, Z₁⁻ dérive d'un acide de pKa inférieur à 4, mieux encore inférieur à 2. Les pKa dont il est question ici sont les pKa des acides tels que mesurés dans l'eau.

Des exemples d'acides sont les acides carboxyliques de formule :
Gₒ-COOH, dans laquelle Gₒ représente alkyle, et par exemple (C1-C22)alkyle ; ou bien aryle, et par exemple (C6-C18)aryle éventuellement substitué par un ou plusieurs alkyle, de préférence un ou plusieurs (C1-C6)alkyle ; les acides sulfoniques de formule : Gₒ-SO₃H, dans laquelle Gₒ est tel que défini ci-dessus ; et les acides phosphoniques de formule : Gₒ-PO₃H dans laquelle Gₒ est tel que défini ci-dessus ; d'autres acides sont HF, HCl, HBr, HI, H₂SO₄, H₃PO₄, HClO₄ et HBF₄.

Des exemples préférés d'acides carboxyliques sont l'acide acétique, l'acide benzoïque, et l'acide stéarique. A titre d'acide sulfonique préféré, on citera l'acide benzène sulfonique et à titre d'acide phosphonique préféré, on mentionnera l'acide phénylphosphonique.

Selon l'invention, on préfère plus particulièrement les anions Z₁⁻ dérivés des acides HCl, HI et HBF₄ et HPF₆.

Ainsi, des anions Z₁⁻ particulièrement préférés, selon l'invention, sont les anions halogénure et le tétrafluoroborate et hexafluorophosphate.
On donne ci-dessous quelques exemples de sels d'imidazolium. 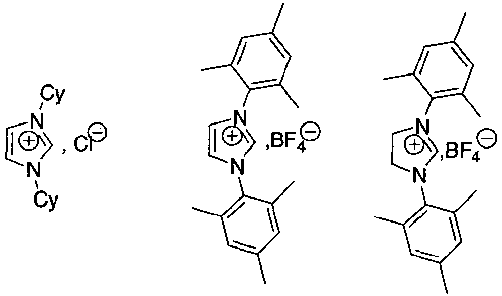 Ces consommables sont soit disponibles dans le commerce, soit facilement préparés par l'homme du métier à partir de composés commerciaux.

Une méthode de synthèse des sels de formule (III) dans laquelle A = B = C est décrite dans US-B-5 077 414.

Ce procédé comprend la réaction :
⇒ d'un composé a-dicarbonylé de formule (X) suivante : 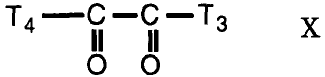 dans laquelle T3 et T4 sont tels que définis ci-dessus
⇒ avec HCHO et deux amines de formules T1-NH₂ et T2-NH₂, en présence d'un acide approprié.

La nature de l'anion Z1 dans les sels de formule (III) dépend de l'acide utilisé à cette étape. Les acides utilisables sont par exemple ceux énumérés ci-dessus et ceux dont dérive Z1.

D'autres méthodes de préparation des sels de formule (III) sont proposées dans Chem. Eur. J. 1996, 2, n° 12, pages 1627-1636 et Angew. Chem. Int. Ed. Engl. 1997, 36, 2162-2187.

De préférence, l'alcool répond à la formule (I°) R°OH, dans laquelle R° correspond à un groupement alkyle; cycloalkyle; aryle; alcényle; alcynyle; arylalkyle; silyle ou siloxane, R° étant de préférence choisi dans le groupe comprenant : méthyle, éthyle, propyle, butyle. La base forte selon l'invention choisie dans le groupe des hydroxydes, des alcoolates, des hydrures ou des amidures alcalins ou alcalinoterreux, de préférence dans le sous-groupe comprenant : CH₃ONa, tertiobutylate de potassium, KOH, NaOH, CH₃CH₂OMgOCH₂CH₃ et leurs mélanges.

Selon une éventualité, cette base forte peut se former *in situ,* par exemple par ajout de sodium, ou d'hydrure de sodium, ou de magnésium dans l'alcool considéré. C'est ainsi que les alcoolates pouvant être générés in situ. Conformément à l'invention, la déprotonation produit un sel qui précipite et que l'on sépare de la solution **A,**

De préférence, le solvant est sublimé de manière à recueillir le carbène **C** sous forme solide.

L'invention concerne aussi une solution A obtenue par le procédé tel que décrit ci-dessus, caractérisée en ce qu'elle est à base d'alcool et d'au moins un carbène **C** de formule (II) ou (II') : 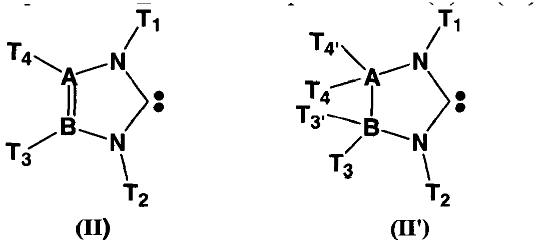 dans lesquelles :
- A et B représentent indépendamment C ou N, étant entendu que :
   - dans la formule (II), lorsque A représente N, alors T4 n'est pas présent et lorsque B représente N, alors T3 n'est pas présent ;
   - dans la formule (II'), lorsque A représente N, alors T4 ou T4' n'est pas présent et lorsque B représente N, alors T3 ou T3' n'est pas présent ;
- T3, T3', T4 et T4' représentent indépendamment un atome d'hydrogène ; un groupement alkyle ; cycloalkyle éventuellement substitué par alkyle ou alkoxy ; aryle éventuellement substitué par alkyle ou alkoxy ; alcényle ; alcynyle ; ou arylalkyle dans lequel la partie aryle est éventuellement substituée par alkyle ou alkoxy ; ou bien
- T3 et T4 peuvent former ensemble et avec A et B quand ceux-ci représentent chacun un atome de carbone, un aryle étant entendu que dans ce cas T3' et T4' ne sont pas présents ;
- T1 et T2 représentent indépendamment un groupement alkyle ; un groupement alkyle éventuellement substitué par alkyle; un groupement alkyle perfluoré ou éventuellement substitué par un groupement perfluoroalkyle ; cycloalkyle éventuellement substitué par alkyle ou alkoxy; aryle éventuellement substitué par alkyle ou alkoxy ; alcényle ; alcynyle ; ou arylalkyle dans lequel la partie aryle est éventuellement substituée par alkyle ou alkoxy ; ou bien
- T1 et T2 représentent indépendamment un radical monovalent de formule (III) suivante :

   -V1-V2 (III)

   dans laquelle :
   - V1 est un groupement divalent hydrocarboné saturé ou non, de préférence un alkylène linéaire ou ramifié en C1-C10, éventuellement substitué,
   - V2 est un groupement monovalent choisi dans le groupe des substituants suivants :
      ◆ alcoxy, -OR^{a} avec R^{a} correspondant à hydrogène, alkyle, aryle ;
      ◆ silyl, -Si(OR^{b})ₓ(R^{c})₃₋ₓ avec R^{b} correspondant à hydrogène, alkyle, silyle ou siloxanyle, R^{c} correspondant à alkyle, aryle et x étant un entier compris entre 0 et 3 ;
      ◆ amine, de préférence -N(R^{a})₂ avec R^{a} correspondant à hydrogène, alkyle, aryle ; ou bien encore ;
- les substituants T1, T2, T3, T3' T4 et T4' peuvent former deux à deux, lorsqu'ils sont situés sur deux sommets adjacents dans les formules (II) et (II'), une chaîne hydrocarbonée saturée ou insaturée.

Par alkyle, on désigne une chaîne hydrocarbonée saturée, linéaire ou ramifiée, éventuellement substituée (e.g. par un ou plusieurs alkyles), de préférence de 1 à 10 atomes de carbone, par exemple de 1 à 8 atomes de carbone, mieux encore de 1 à 7 atomes de carbone.

Des exemples de groupements alkyle sont notamment méthyle, éthyle, isopropyle, n-propyle, tert-butyle, isobutyle, n-butyle, n-pentyle, isoamyle et 1,1-diméthylpropyle.
La partie alkyle du groupement alcoxy est telle que définie ci-dessus.
Le groupement alkyle perfluoré ou éventuellement substitué par un groupement perfluoroalkyle correspond, de préférence, à la formule :

**-(CH₂)ₚ-C_{q}F_{2q+1}**

dans laquelle p représente 0, 1, 2, 3 ou 4; q est un entier de 1 à 10 ; et C_{q}F_{2q+1} est linéaire ou ramifié. Des exemples préférés de ce groupement sont : -(CH₂)₂-(CF₂)₅-CF₃ et -(CF₂)₇-CF₃.

L'expression aryle désigne un groupement hydrocarboné aromatique, ayant de 6 à 18 atomes de carbone, monocyclique ou polycyclique et de préférence monocyclique ou bicyclique. Il doit être entendu que, dans le cadre de l'invention, par groupement aromatique polycyclique, on entend un groupement présentant deux ou plusieurs noyaux aromatiques, condensés (orthocondensés ou ortho et péricondensés) les uns aux autres, c'est-à-dire présentant, deux à deux, au moins deux carbones en commun.
Ledit groupement hydrocarboné aromatique ("aryle") est éventuellement substitué par exemple par un ou plusieurs alkyles en C1-C3, un ou plusieurs groupements hydrocarbonés halogénés (e.g. CF₃), un ou plusieurs alcoxy (e.g. CH₃O) ou un ou plusieurs groupements hydrocarbonés comprenant un ou plusieurs motifs cétone (e.g. CH₃CO-).
A titre d'exemple d'aryle, on peut mentionner les radicaux phényle, naphtyle, anthryle et phénanthryle.

L'expression arylalkyle désigne un groupement alkyle tel que défini ci-dessus, substitué par un ou plusieurs groupements aryle sur sa chaîne hydrocarbonée, le groupement aryle étant tel que défini ci-dessus. Des exemples en sont benzyle et triphénylméthyle.

Par cycloalkyle, on entend un groupement hydrocarboné saturé mono- ou polycyclique, de préférence mono- ou bicyclique, présentant préférablement de 3 à 10 atomes de carbone, mieux encore de 3 à 8. Par groupement hydrocarboné saturé polycyclique, on entend un groupement présentant deux ou plusieurs noyaux cycliques rattachés les uns aux autres par des liaisons σ ou/et condensés deux à deux.
Des exemples de groupements cycloalkyle polycycliques sont adamantane et norbornane. Des exemples de groupements cycloalkyle monocycliques sont cyclopropyle, cyclobutyle, cyclopentyle, cyclohexyle, cycloheptyle et cyclooctyle.

Par alcényle, on entend une chaîne hydrocarbonée insaturée, linéaire ou ramifiée, substituée ou non, présentant au moins une double liaison oléfinique, et plus préférablement une seule double liaison. De préférence, le groupement alcényle présente de 2 à 8 atomes de carbone, mieux encore de 2 à 6. Cette chaîne hydrocarbonée comprend éventuellement au moins un hétéroatome tel que O, N, S.
Des exemples préférés de groupements alcényle sont les groupements allyle et homoallyle. Par alcynyle, on entend selon l'invention, une chaîne hydrocarbonée insaturée, linéaire ou ramifiée, substituée ou non, présentant au moins une triple liaison acétylénique, et plus préférablement une seule triple liaison. De préference, le groupement alcynyle présente de 2 à 8 atomes de carbone, mieux encore de 2 à 6 atomes de carbone. A titre d'exemple, on peut citer le groupement acétylényle, ainsi que le groupement propargyle. Cette chaîne hydrocarbonée comprend éventuellement au moins un hétéroatome tel que O, N, S.

Par silyle, on entend selon l'invention, un groupement linéaire ou ramifié contenant au moins un atome de silicium. Les chaînes polydiméthylsiloxane sont des exemples de groupements silyle.

Les carbènes de formule (**II**) et (**II'**) peuvent présenter au moins deux noyaux condensés, c'est-à-dire que deux groupements au moins parmi T1, T2, T3, T3', T4 et T4' situés sur deux sommets adjacents, forment ensemble une chaîne hydrocarbonée saturée ou insaturée, présentant de préférence de 3 à 6 atomes de carbones. Par chaîne hydrocarbonée saturée ou insaturée, on entend une chaîne hydrocarbonée linéaire ou ramifiée pouvant présenter ou non une ou plusieurs insaturations de type double liaison oléfinique ou triple liaison acétylénique.

Concernant les formes préférées de réalisation dans les formules (II) ou (II'), ce sont des formes dans lesquelles A = B = atome de carbone dans la formule (II) donnée supra.

Des significations préférées pour T1 et T2 dans cette formule (II) sont :
- alkyle, en particulier n-propyle, n-pentyle, néo-pentyle (-CH₂-C(CH₃)₃) ;
- cycloalkyle, en particulier cyclopentyle, cyclohexyle ou adamantyle ;
- alcényle, en particulier allyle (-CH₂-CH=CH₂), méthallyle (-CH₂-C(CH₃)=CH₂) ;
- alcynyle, en particulier propargyle, homopropargyle (-(CH₂)₂-C≡CH) ;
- ou groupement monovalent (V) défini supra, en particulier :

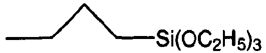

Toujours dans la formule (**II**) et de préférence, T3 et T4 correspondent tous deux à l'hydrogène ou forment ensemble un aryle, et mieux encore un phényle.

A titre d'exemples de carbènes, on peut citer ceux décrits dans le tableau 2, page 48 de la publication "Bourissou et al. Chem. Rev. 2000, 100, 39-91".

Conformément à l'invention, le (ou les) carbène(s) :
- est (sont) préparé(s) séparément,
- et/ou est (sont) généré(s) in-situ à partir d'au moins un précurseur.

### La nouvelle forme liquide ou solide de carbène

Le procédé selon l'invention donne donc accès à une solution à base d'alcool et de carbène. De manière surprenante et inattendue, cette solution alcoolique est stable et catalytique.

L'invention couvre également cette solution alcoolique stable et catalytique de carbène *per se,* obtenue par le procédé selon l'invention.

### Les applications

Suivant un autre de ses aspects, le présent exposé vise un procédé de préparation de PolyOrganoSiloxanes (POS) par polymérisation par ouverture de cycle et/ou par redistribution de POS et/ou par polycondensation, en présence d'un catalyseur (C) comprenant au moins un carbène tel qu'obtenu par le procédé tel que défini supra, ledit carbène se présentant sous la forme d'une solution alcoolique ou d'un solide extrait dudit liquide.

Au sens du présent exposé, la "polymérisation par ouverture de cycle(s)" correspond à une polymérisation dans laquelle un composé cyclique (monomère) est ouvert pour former un polymère linéaire.

Au sens du présent exposé, la "polymérisation par redistribution" correspond à l'acception de l'homme du métier dans la chimie des silicones. En particulier, la "polymérisation par redistribution" s'entend dans le domaine des silicones, comme un réarrangement d'organosiloxanes de structures et/ou de masse molaire différentes. Ce réarrangement conduit à un seul nouveau POS.

L'invention ouvre de nouvelles voies dans la catalyse de :
- la préparation de PolyOrganoSiloxanes (POS) par polymérisation par ouverture de cycle et/ou par redistribution et/ou polycondensation de POS;
- l'hydrosilylation d'au moins un PolyOrganoSiloxane -A- (POS) porteur d'insaturation(s) éthylènique(s) et/ou acétylènique(s), à l'aide d'au moins un polyorganohydrogénosiloxane -B-, ou d'autres composés éthyléniquement et/ou acétyléniquement insaturés ;
- l'hydrogénation ;
- la métathèse d'oléfines ;
- le couplage croisé C-C et/ou C-N ;
- la synthèse asymétrique ou énantiosélective ;
- ou la synthèse organique : réactions d'estérification, trans-estérification ; condensation de type aldolisation, par exemple.

Ces voies sont performantes en termes de stockage et de manipulation de facilité de mise en oeuvre du catalyseur au carbène et en termes d'activité catalytique, de sélectivité (meilleurs taux de transformation et rendement), de facilité de mise en oeuvre (basse température, pas ou peu de purification), d'obtention de POS ayant de bons indices de polymolécularité et pouvant avoir de fortes viscosités, entre autres.

Ces performances sont d'autant plus intéressantes qu'elles se doublent d'avantages en termes de coût, de sécurité et de non écotoxicité, notamment.

Les exemples qui suivent permettront de mieux appréhender le procédé et le carbène liquide ou solide selon l'invention, en faisant ressortir tous leurs avantages et les variantes possibles de mises en oeuvre.

### EXEMPLES

### Généralités :

Dans le protocole suivant l'invention, on utilise directement un alcool comme solvant de la réaction, permettant la protection instantanée du carbène dès sa formation. La synthèse est réalisée à l'air libre.

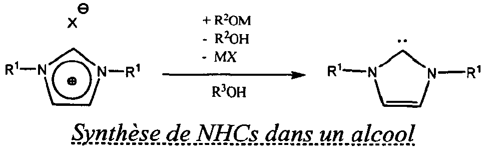

Cette synthèse d'un NHC (R¹: tert-butyle) a été testée dans plusieurs conditions par variation de la base et de l'alcool utilisés. Il en ressort que l'utilisation d'un solvant de type éthanol ou isopropanol et d'une base de type tBuOK ou KOH permettent l'obtention du carbène. En effet les spectres RMN (proton et carbone) des solutions sont identiques à ceux obtenus dans le cas d'un mélange, de carbène pur et d'alcool.

Les éléments permettant d'attester la formation du carbène sont notamment :
- L'absence de proton sur le carbone situé entre les deux atomes d'azote (spectre RMN ¹H)
- La présence d'un signal à 130 ppm correspondant à un carbone de type quaternaire (spectre RMN ¹³C)

Le procédé selon l'invention a ainsi permis de synthétiser un nouveau carbène, habituellement trop instable pour être isolé (R¹: méthyle).

### -A- Synthèse

### Exemple A1 : Synthèse du NHC-tBu par déprotonation avec tBuOK dans l'isopropanol

On pèse 210 mg de tBuOK (1,87 mmole) auxquels on rajoute à l'air libre une solution de 500 mg de sel d'imidazolium (1,87 mmole) dans environ 4 mL d'isopropanol sous agitation. On observe une légère exothermie dans les premières minutes. Après environ 1h d'agitation à température ambiante on élimine KCl par filtration sur fritté et on obtient une solution légèrement colorée.

Les spectres RMN (proton et carbone) de cette solution sont représentés ci-dessous.

### Exemple A2 : Synthèse du NHC-tBu par déprotonation avec KOH dans l'isopropanol

On pèse 93 mg de KOH en pastille (1,66 mmole) que l'on dissout dans 2 mL d'isopropanol, en rajoutant quelques gouttes d'eau distillée afin de bien dissoudre. On rajoute ensuite 450 mg de sel d'imidazolium (1,66 mmole) dissouts dans 2 mL d'isopropanol. On observe une légère exothermie dans les premières minutes. Après environ 1h d'agitation à température ambiante on filtre sur fritté et on obtient une solution légèrement colorée.

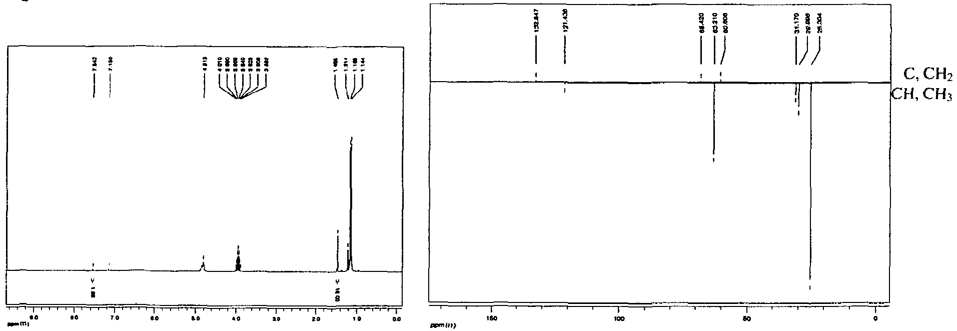

### Spectre 1 : RMN ¹H et ¹³C (synthèse du NHC-tBu, base : tBuOK, solvant : iPrOH)

| **RMN ¹H** | **RMN ¹³C (jmod)** |
|---|---|
| 1.15 (iPrOH) | 25.3 (iPrOH) |
| 1.21 (tBuOH) | **30.0 (N-C-(CH₃)₃)** |
| **1.47 (s, 18H, N-C-(CH₃)₃)** | 31.2 (tBuOH) |
| 3.95 (iPrOH) | **60.6 (N-C-(CH₃)₃)** |
| 4.81 (iPrOH) | 63.2 (iPrOH) |
| 7.15 (C₆D₆) | 68.4 (tBuOH) |
| **7.54 (s, 2H, N-CH=CH-N)** | **121.4 (N-CH=CH-N)** |
| | **132.8 (C carbénique)** |

### Exemple A3 : Préparation d'une solution éthanolique du carbène NHC-Cy2 (Cy = cyclohexyl) :

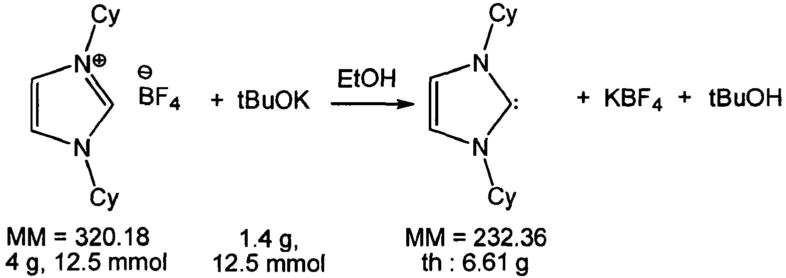

Dans un ballon de 50 ml contenant 4 g de tétrafluoroborate de N,N'-dicyclohexylimidazolium (12.5 mmol) et 1.4 g de tertiobutylate de potassium (12.5 mmol) refroidis à 0°C est ajouté 20 ml d'éthanol. Après 10 min, le mélange hétérogène est agité à 20°C pendant une à deux heures. Le solide (KBF4) est alors filtré, puis rincé par 6 ml d'éthanol. Le filtrat limpide légèrement jaune est concentré pour donner une solution de carbène à 43.87 %p théorique, utilisée telle quelle comme catalyseur ou complexant. Il est préférable de conserver cette solution à température ambiante (20°C), le carbène pouvant cristalliser à froid. Un gaz inerte n'est pas nécessaire.

### -B- Réactivité

### B1 Catalyseurs d'hydrosilylation : platine-carbène

### B11 Synthèse de « platine-carbène » :

### Réaction générale :

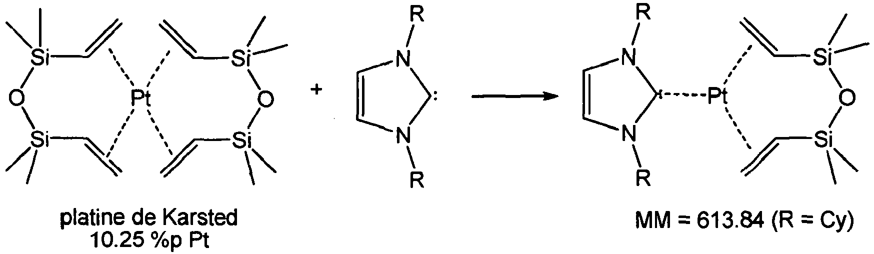

### Exemple B111 : Synthèse à partir de tétrafluoroborate de dicyclohexylimidazolium (WO 02/98888 du 4 juin 2002) → échantillon 1

La synthèse nécessite un réacteur sous gaz inerte, du THF anhydre, du toluène pour précipiter la majeure partie du sel de potassium, 2 filtrations ; la productivité est faible (13 % p/p), le rendement moyen (87%) et le produit isolé contient le plus souvent des sels (dosage Pt = 30.4 %p pour une théorie de 31.8 %p).

### Exemple B112 : Synthèse en milieu dilué THF selon l'invention (dilution égale à 1)) -→ échantillon 2

Dans un ballon monocol de 50 ml sont placés 10 g de platine de Karsted à 10.25 %p de platine (5.25 mmol) et 10 ml de THF. A la solution obtenue est ajouté sous agitation 3.06 g de la solution de carbène NHC-Cy2 décrite en A3 (5.78 mmol). Après 2h à 20°C, la solution est évaporée, le solide gras est repris par 20 ml d'isopropanol, la suspension est filtrée et le solide est séché sous vide : 2.8 g obtenus soit 87.5 % de rendement.
Anal. élém. : 31.01 % Pt
→ rendement non optimisé égal, produit plus pur, procédé très simplifié.

### Exemple B113 : Synthèse sans solvant → échantillon 3

Même mode opératoire que ci-dessus sans THF : ajout de la solution de carbène sur le platine de Karsted, précipitation après 10 min d'agitation. Après 2h, 20 ml d'isopropanol sont ajouté. Le solide blanc est filtré, rincé par 5 ml du même solvant et séché sous vide : 2.75 g obtenus soit rendement 86 %.
Anal. élém. : 31.43 % Pt
→ rendement non optimisé égal, produit encore plus pur, procédé encore simplifié.

### Exemple B114 : Même réaction que B112 (avec THF) avec une solution de carbène préparée un mois auparavant et stockée sans précaution à 20°C → échantillon 4

2.8 g obtenus soit 87.5 % de rendement.
Anal. élém. : 31.51 %Pt
→ rendement non optimisé égal, produit encore plus pur, procédé robuste et simple.

### Exemple B115 : Préparation du platine carbène avec carbène = NHC-tBu2 :

Même mode opératoire que dans l'exemple B112, avec une solution éthanolique de carbène NHC-tBu2 à 37.8 %p (2.76 g pour 5.78 mmol). Obtention de 2.6 g d'un solide blanc fin soit rendement 88 %. Anal. élém. : 34.56 % Pt (th. 34.73 %)

### Exemple B12 : Tests des catalyseurs

La réaction étudiée est l'hydrosilylation du 1-octène par le 1,1,1,2,3,3,3-heptaméthyltrisiloxane sans solvant :

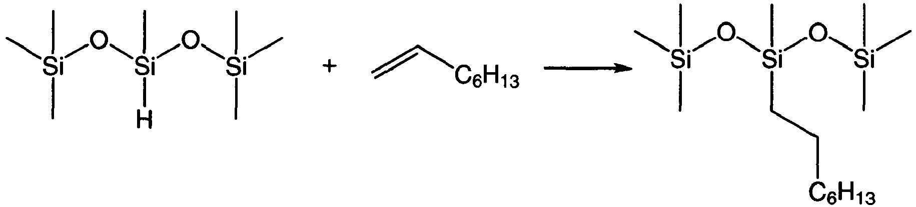

Dans un ballon tricol de 50 ml sont placés 10 g d'octène (89.1 mmol, 1.2 éq). Le ballon est chauffé à 85°C en consigne. 27 µl de solution de platine carbène à 2.7 % dans le xylène (1.1 x 10-3 mol%/octène) sont ajoutés et l'hydrosiloxane est ajouté à l'aide d'un pousse seringue sur 1h30. La disparition de l'hydrosiloxane est suivie au cours du temps par gazométrie. Les résultats de conversion de l'hydrosilane sont donnés dans le tableau ci-dessous :

| Pt-carbène | conversion SiH à 20% de coulée | conversion SiH à 50% de coulée | conversion SiH à 100% de coulée | 1h30 après fin coulée |
|---|---|---|---|---|
| échantillon 1 | 11,0% | 76,0% | 90,0% | 99,7% |
| échantillon 2 | 8,3% | 70,0% | 91,0% | 99,5% |
| échantillon 3 | 10,0% | 75,0% | 92,0% | 99,7% |
| échantillon 4 | 16,7% | 78,0% | 91,0% | 99,8% |

On en déduit que tous les catalyseurs sont globalement équivalents.

### Exemple B13 :

On évalue la stabilité en solution d'un carbène NHC di tertiobutyl (NHC-tBu2, préparé à l'Exemple B115) :
a) Comparatif, dans le THF et sous atmosphère d'air ambiant après :
   - 3h : 0% de dégradation,
   - 20h : 2% de dégradation,
   - 48h: 25% de dégradation,
      - 7 jours : 100% de dégradation
b) Invention, en solution (40% poids) dans MeOH, EtOH ou iPrOH :
   - après 1 an aucune dégradation

## Revendications

1. Procédé de préparation d'un catalyseur ou d'un réactif comprenant un carbène et stable à 20° C se présentant sous la forme d'une solution alcoolique **A** stable constituée d'au moins un alcool et d'au moins un carbène comprenant les étapes suivantes :
- on fait réagir dans un solvant qui est un alcool ou un mélange d'alcools, au moins une base forte, éventuellement générée in situ, avec au moins un composé **B** précurseur d'un carbène **C,** ledit composé **B** qui est un sel comprenant une fonction iminium est choisi parmi les composés de formule générale (III) ou (III') suivante : 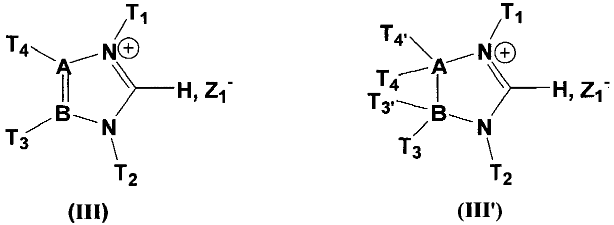 dans laquelle :
- A et B représentent indépendamment C ou N, étant entendu que :
• dans la formule (III), lorsque A représente N, alors T4 n'est pas présent et lorsque B représente N, alors T3 n'est pas présent ;
• dans la formule (III'), lorsque A représente N, alors T4 ou T4' n'est pas présent et lorsque B représente N, alors T3 ou T3' n'est pas présent ;
- T3, T3', T4 et T4' représentent indépendamment un atome d'hydrogène; un groupement alkyle ; cycloalkyle éventuellement substitué par un groupe alkyle ou alkoxy ; aryle éventuellement substitué par un groupe alkyle ou alkoxy ; alcényle ; alcynyle ; ou arylalkyle dans lequel la partie aryle est éventuellement substituée par un groupe alkyle ou alkoxy ; ou bien
- T3 et T4 peuvent former ensemble et avec A et B quand ceux-ci représentent chacun un atome de carbone, un aryle étant entendu que dans ce cas T3' et T4' ne sont pas présents ;
- T1 et T2 représentent indépendamment un groupement alkyle ; un groupement alkyle éventuellement substitué par un alkyle ; un groupement alkyle perfluoré ou éventuellement substitué par un groupement perfluoroalkyle ; cycloalkyle éventuellement substitué par un groupe alkyle ou alkoxy ; aryle éventuellement substitué par un groupe alkyle ou alkoxy ; alcényle ; alcynyle ; ou arylalkyle dans lequel la partie aryle est éventuellement substituée par un groupe alkyle ou alkoxy ; ou bien
- T1 et T2 représentent indépendamment un radical monovalent de formule (III) suivante :
-V1-V2 (III)
dans laquelle :
• V1 est un groupement divalent hydrocarboné saturé ou non, éventuellement substitué,
• V2 est un groupement monovalent choisi dans le groupe des substituants suivants :
◆ alcoxy, -OR^{a} avec R^{a} correspondant à hydrogène, alkyle, aryle ;
◆ silyl, -Si(OR^{b})ₓ(R^{c})₃₋ₓ avec R^{b} correspondant à hydrogène, alkyle, silyle ou siloxanyle, R^{c} correspondant à alkyle, aryle et x étant un entier compris entre 0 et 3 ;
◆ amine, ou bien encore ;
- les substituants T1, T2, T3, T3' T4 et T4' peuvent former deux à deux, lorsqu'ils sont situés sur deux sommets adjacents dans les formules (III) et (III'), une chaîne hydrocarbonée saturée ou insaturée ;
- Z1 représente indépendamment un anion dérivé d'un acide de Brönsted (acide protique)
avec comme conditions suivantes :
- le choix de la base forte s'effectue de manière à pouvoir effectuer une déprotonation de l'hydrogène de la fonction iminium de structure (III) ou (III') et est choisie dans le groupe des hydroxydes, des alcoolates, des hydrures ou des amidures alcalins ou alcalinoterreux,
- la réaction s'effectue dans un solvant qui est un alcool ou un mélange d' alcools, et
- la déprotonation produit un sel qui précipite et que l'on sépare de la solution **A,**

2. Procédé selon la revendication 1, **caractérisé en ce que** l'alcool répond à la formule (I°) R°OH, dans laquelle R° correspond à un groupement alkyle; cycloalkyle; aryle; alcényle; alcynyle; arylalkyle; silyle ou siloxane.

3. Solution alcoolique **A, caractérisé en ce qu'**elle est obtenue selon le procédé tel que décrit selon l'une quelconque des revendications 1 à 2 et constituée de :
• un solvant qui est un alcool ou un mélange d' alcools,
• et d'au moins un carbène **C** qui a pour formule (II) ou (II') : 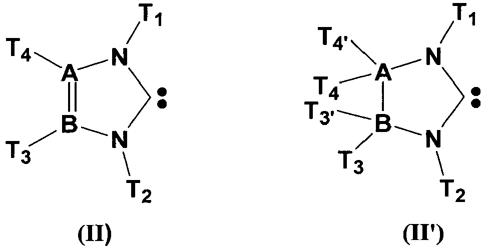 dans lesquelles :
- A et B représentent indépendamment C ou N, étant entendu que :
• dans la formule (II), lorsque A représente N, alors T4 n'est pas présent et lorsque B représente N, alors T3 n'est pas présent ;
• dans la formule (II'), lorsque A représente N, alors T4 ou T4' n'est pas présent et lorsque B représente N, alors T3 ou T3' n'est pas présent ;
- T3, T3', T4 et T4' représentent indépendamment un atome d'hydrogène ; un groupement alkyle ; cycloalkyle éventuellement substitué par alkyle ou alkoxy ; aryle éventuellement substitué par alkyle ou alkoxy ; alcényle ; alcynyle ; ou arylalkyle dans lequel la partie aryle est éventuellement substituée par alkyle ou alkoxy ; ou bien
- T3 et T4 peuvent former ensemble et avec A et B quand ceux-ci représentent chacun un atome de carbone, un aryle étant entendu que dans ce cas T3' et T4' ne sont pas présents ;
- T1 et T2 représentent indépendamment un groupement alkyle ; un groupement alkyle éventuellement substitué par alkyle ; un groupement alkyle perfluoré ou éventuellement substitué par un groupement perfluoroalkyle ; cycloalkyle éventuellement substitué par alkyle ou alkoxy ; aryle éventuellement substitué par alkyle ou alkoxy ; alcényle ; alcynyle ; ou arylalkyle dans lequel la partie aryle est éventuellement substituée par alkyle ou alkoxy ; ou bien
- T1 et T2 représentent indépendamment un radical monovalent de formule (III) suivante :
-V1-V2 (III)
dans laquelle :
• V1 est un groupement divalent hydrocarboné saturé ou non,
• V2 est un groupement monovalent choisi dans le groupe des substituants suivants :
◆ alcoxy, -OR^{a} avec R^{a} correspondant à hydrogène, alkyle, aryle ;
◆ silyl, -Si(OR^{b})ₓ(R^{c})₃₋ₓ avec R^{b} correspondant à hydrogène, alkyle, silyle ou siloxanyle, R^{c} correspondant à alkyle, aryle et x étant un entier compris entre 0 et 3 ;
◆ amine, alkyle, aryle ; ou bien encore ;
- les substituants T1, T2, T3, T3' T4 et T4' peuvent former deux à deux, lorsqu'ils sont situés sur deux sommets adjacents dans les formules (II) et (II'), une chaîne hydrocarbonée saturée ou insaturée.

## Patentansprüche

1. Verfahren zur Herstellung eines Katalysators oder eines Reaktanten, der ein Carben umfasst, bei 20 °C stabil ist und in Form einer stabilen alkoholischen Lösung **A,** die aus mindestens einem Alkohol und mindestens einem Carben besteht, vorliegt, das folgende Schritte umfasst:
- man setzt in einem Lösungsmittel, bei dem es sich um einen Alkohol oder eine Mischung von Alkoholen handelt, mindestens eine starke Base, die gegebenenfalls in situ erzeugt wird, mit mindestens einer Verbindung **B,** bei der es sich um die Vorstufe eines Carbens **C** handelt, um, die Verbindung **B,** bei der es sich um ein Salz handelt, das eine Iminium-Funktion umfasst, aus den Verbindungen der folgenden allgemeinen Formel (III) oder (III') ausgewählt wird: 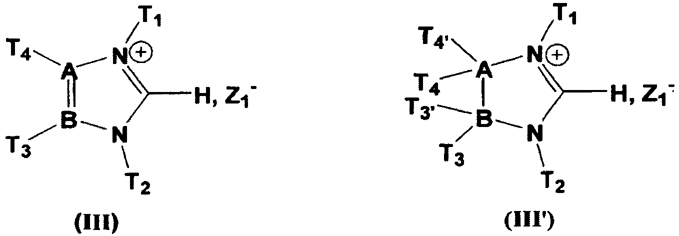 wobei:
- A und B unabhängig voneinander für C oder N stehen, wobei es sich versteht, dass:
• in der Formel (III) dann, wenn A für N steht, T4 nicht vorhanden ist, und dann, wenn B für N steht, T3 nicht vorhanden ist;
• in der Formel (III') dann, wenn A für N steht, T4 oder T4' nicht vorhanden ist, und dann, wenn B für N steht, T3 oder T3' nicht vorhanden ist;
- T3, T3', T4 und T4' unabhängig für ein Wasserstoffatom; eine Alkylgruppe; eine Cycloalkylgruppe, die gegebenenfalls durch eine Alkyl- oder Alkoxygruppe substituiert ist; eine Arylgruppe, die gegebenenfalls durch eine Alkyl- oder Alkoxygruppe substituiert ist; eine Alkenylgruppe; eine Alkinylgruppe oder eine Arylalkylgruppe, in der der Arylteil gegebenenfalls durch eine Alkyl- oder Alkoxygruppe substituiert ist; stehen oder auch
- T3 und T4 zusammen und mit A und B, wenn diese jeweils für ein Kohlenstoffatom stehen, ein Aryl bilden können, wobei es sich versteht, dass in diesem Fall T3' und T4' nicht vorhanden sind;
- T1 und T2 unabhängig für eine Alkylgruppe; eine Alkylgruppe, die gegebenenfalls durch ein Alkyl substituiert ist; eine Alkylgruppe, die perfluoriert oder gegebenenfalls durch eine Perfluoralkylgruppe substituiert ist; eine Cycloalkylgruppe, die gegebenenfalls durch eine Alkyl- oder Alkoxygruppe substituiert ist; eine Arylgruppe, die gegebenenfalls durch eine Alkyl- oder Alkoxygruppe substituiert ist; eine Alkenylgruppe; eine Alkinylgruppe oder eine Arylalkylgruppe, in der der Arylteil gegebenenfalls durch eine Alkyl- oder Alkoxygruppe substituiert ist; stehen oder auch
- T1 und T2 unabhängig für einen einwertigen Rest der folgenden Formel (III) stehen:
**-V1-V2** **(III)**
wobei:
• V1 für eine gesättigte oder ungesättigte zweiwertige Kohlenwasserstoffgruppe, die gegebenenfalls substituiert ist, steht,
• V2 für eine einwertige Gruppe steht, die aus der Gruppe der folgenden Substituenten ausgewählt ist:
◆ Alkoxy, -OR^{a}, wobei R^{a} für Wasserstoff, Alkyl oder Aryl steht;
◆ Silyl, -Si (OR^{b})ₓ(R^{c})₃₋ₓ, wobei R^{b} Wasserstoff, Alkyl, Silyl oder Siloxanyl entspricht, R^{c} Alkyl oder Aryl entspricht und x für eine ganze Zahl zwischen 0 und 3 steht;
◆ Amin, oder auch
- die Substituenten T1, T2, T3, T3', T4 und T4' dann, wenn sie sich in den Formeln (III) und (III') an zwei benachbarten Spitzen befinden, paarweise eine gesättigte oder ungesättigte Kohlenwasserstoffkette bilden können;
- Z1 unabhängig für ein Anion, das sich von einer Brönsted-Säure (Protonensäure) ableitet, steht;
mit den folgenden Bedingungen:
- die Wahl der starken Base wird so vorgenommen, dass eine Deprotonierung des Wasserstoffs der Iminium-Funktion der Struktur (III) oder (III') durchgeführt werden kann, und wird aus der Gruppe der Alkali- oder Erdalkalihydroxide, -alkoholate, -hydride oder -amide ausgewählt,
- die Umsetzung wird in einem Lösungsmittel durchgeführt, bei dem es sich um einen Alkohol oder ein Gemisch von Alkoholen handelt, und
- die Deprotonierung ergibt ein Salz, das ausfällt und von der Lösung **A** abgetrennt wird.

2. Verfahren nach Anspruch 1, **dadurch gekennzeichnet, dass** der Alkohol der Formel (I°) R°OH entspricht, wobei R° einer Alkyl-, Cycloalkyl-, Aryl-, Alkenyl-, Alkinyl-, Arylalkyl-, Silyl- oder Siloxangruppe entspricht.

3. Alkoholische Lösung **A, dadurch gekennzeichnet, dass** sie nach dem Verfahren gemäß einem der Ansprüche 1 bis 2 erhalten wird und aus:
• einem Lösungsmittel, bei dem es sich um einen Alkohol oder eine Mischung von Alkoholen handelt,
• und mindestens einem Carben **C** der Formel (II) oder (II') : 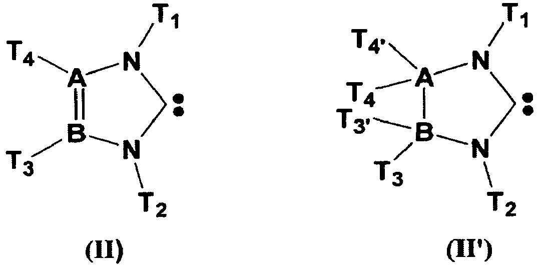 besteht,
wobei:
- A und B unabhängig voneinander für C oder N stehen, wobei es sich versteht, dass:
• in der Formel (II) dann, wenn A für N steht, T4 nicht vorhanden ist, und dann, wenn B für N steht, T3 nicht vorhanden ist;
• in der Formel (II') dann, wenn A für N steht, T4 oder T4' nicht vorhanden ist, und dann, wenn B für N steht, T3 oder T3' nicht vorhanden ist;
- T3, T3', T4 und T4' unabhängig für ein Wasserstoffatom; eine Alkylgruppe; eine Cycloalkylgruppe, die gegebenenfalls durch eine Alkyl- oder Alkoxygruppe substituiert ist; eine Arylgruppe, die gegebenenfalls durch eine Alkyl- oder Alkoxygruppe substituiert ist; eine Alkenylgruppe; eine Alkinylgruppe oder eine Arylalkylgruppe, in der der Arylteil gegebenenfalls durch eine Alkyl- oder Alkoxygruppe substituiert ist; stehen oder auch
- T3 und T4 zusammen und mit A und B, wenn diese jeweils für ein Kohlenstoffatom stehen, ein Aryl bilden können, wobei es sich versteht, dass in diesem Fall T3' und T4' nicht vorhanden sind;
- T1 und T2 unabhängig für eine Alkylgruppe; eine Alkylgruppe, die gegebenenfalls durch ein Alkyl substituiert ist; eine Alkylgruppe, die perfluoriert oder gegebenenfalls durch eine Perfluoralkylgruppe substituiert ist; eine Cycloalkylgruppe, die gegebenenfalls durch eine Alkyl- oder Alkoxygruppe substituiert ist; eine Arylgruppe, die gegebenenfalls durch eine Alkyl- oder Alkoxygruppe substituiert ist; eine Alkenylgruppe; eine Alkinylgruppe oder eine Arylalkylgruppe, in der der Arylteil gegebenenfalls durch Alkyl oder Alkoxy substituiert ist; stehen oder auch
- T1 und T2 unabhängig für einen einwertigen Rest der folgenden Formel (III) stehen:
**-V1-V2** **(III)**
wobei:
• V1 für eine gesättigte oder ungesättigte zweiwertige Kohlenwasserstoffgruppe steht,
• V2 für eine einwertige Gruppe steht, die aus der Gruppe der folgenden Substituenten ausgewählt ist:
◆ Alkoxy, -OR^{a}, wobei R^{a} für Wasserstoff, Alkyl oder Aryl steht;
◆ Silyl, -Si(OR^{b})ₓ(R^{c})₃₋ₓ, wobei R^{b} Wasserstoff, Alkyl, Silyl oder Siloxanyl entspricht, R^{c} Alkyl oder Aryl entspricht und x für eine ganze Zahl zwischen 0 und 3 steht;
◆ Amin, Alkyl, Aryl; oder auch
- die Substituenten T1, T2, T3, T3', T4 und T4' dann, wenn sie sich in den Formeln (II) und (II') an zwei benachbarten Spitzen befinden, paarweise eine gesättigte oder ungesättigte Kohlenwasserstoffkette bilden können.

## Claims

1. Process for the preparation of a catalyst or of a reactant comprising a carbene and stable at 20°C which is provided in the form of a stable alcoholic solution **A** composed of at least one alcohol and at least one carbene comprising the following stages:
- at least one strong base, optionally generated in situ, is reacted, in a solvent which is an alcohol or a mixture of alcohols, with at least one compound **B** which is the precursor of a carbene **C,** said compound **B,** which is a salt comprising an iminium functional group, is chosen from the compounds of following general formula (III) or (III'): 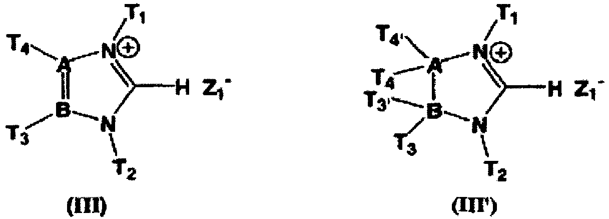 in which:
- A and B independently represent C or N, it being understood that:
• in the formula (III), when A represents N, then T₄ is not present and, when B represents N, then T₃ is not present;
• in the formula (III'), when A represents N, then T₄ or T4' is not present and, when B represents N, then T₃ or T_{3'} is not present;
- T₃, T_{3'}, T₄ and T_{4'} independently represent a hydrogen atom; an alkyl group; a cycloalkyl group optionally substituted by an alkyl or alkoxy group; an aryl group optionally substituted by an alkyl or alkoxy group; an alkenyl group; an alkynyl group; or an arylalkyl group in which the aryl part is optionally substituted by an alkyl or alkoxy group; or else
- T₃ and T₄ can form, together and with A and B when the latter each represent a carbon atom, an aryl, it being understood that, in this case, T_{3'} and T_{4'} are not present;
- T₁ and T₂ independently represent an alkyl group; an alkyl group optionally substituted by an alkyl; an alkyl group which is perfluorinated or optionally substituted by a perfluoroalkyl group; a cycloalkyl group optionally substituted by an alkyl or alkoxy group; an aryl group optionally substituted by an alkyl or alkoxy group; an alkenyl group; an alkynyl group; or an arylalkyl group in which the aryl part is optionally substituted by an alkyl or alkoxy group; or else
- T₁ and T₂ independently represent a monovalent radical of following formula (III):
**-V1-V2** **(III)**
in which:
• V1 is a saturated or unsaturated divalent hydrocarbon group which is optionally substituted,
• V2 is a monovalent group chosen from the group of the following substituents:
◆ alkoxy, -OR^{a}, with R^{a} corresponding to hydrogen, alkyl or aryl;
◆ silyl, -Si (OR^{b})ₓ(R^{c})₃₋ₓ, with R^{b} corresponding to hydrogen, alkyl, silyl or siloxanyl, R^{c} corresponding to alkyl, aryl and x being an integer between 0 and 3;
◆ amine, or alternatively;
- the substituents T₁, T₂, T₃, T_{3'}, T₄ and T4' can form, in pairs, when they are located on two adjacent ring members in the formulae (III) and (III'), a saturated or unsaturated hydrocarbon chain;
- Z₁ independently represents an anion derived from a Bronsted acid (protic acid)
with as following conditions:
- the choice of the strong base is made so as to be able to carry out a deprotonation of the hydrogen of the iminium functional group of structure (III) or (III') and is chosen from the group of the alkali metal and alkaline earth metal hydroxides, alkoxides, hydrides and amides,
- the reaction is carried out in a solvent which is an alcohol or a mixture of alcohols, and the deprotonation produces a salt which precipitates and which is separated from the solution **A.**

2. Process according to Claim 1, **characterized in that** the alcohol corresponds to the formula (I°) R°OH, in which R° corresponds to an alkyl, cycloalkyl, aryl, alkenyl, alkynyl, arylalkyl, silyl or siloxane group.

3. Alcoholic solution **A, characterized in that** it is obtained according to the process as described according to either one of Claims 1 and 2 and consists of: 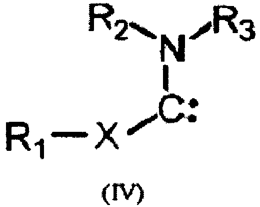
• a solvent which is an alcohol or a mixture of alcohols,
• which has the formula (II) or (II'): 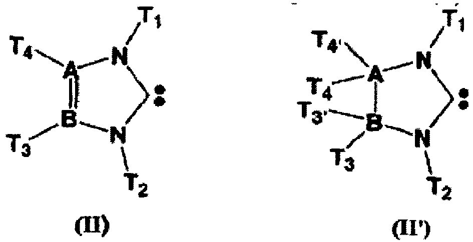 in which:
- A and B independently represent C or N, it being understood that:
• in the formula (II), when A represents N, then T₄ is not present and, when B represents N, then T₃ is not present;
• in the formula (II'), when A represents N, then T₄ or T_{4'} is not present and, when B represents N, then T₃ or T_{3'} is not present;
- T₃, T_{3'}, T₄ and T_{4'} independently represent a hydrogen atom; an alkyl group; a cycloalkyl group optionally substituted by alkyl or alkoxy; an aryl group optionally substituted by alkyl or alkoxy; an alkenyl group; an alkynyl group; or an arylalkyl group in which the aryl part is optionally substituted by alkyl or alkoxy; or else
- T₃ and T₄ can form, together and with A and B when the latter each represent a carbon atom, an aryl, it being understood that, in this case, T_{3'} and T_{4'} are not present;
- T₁ and T₂ independently represent an alkyl group; an alkyl group optionally substituted by alkyl; an alkyl group which is perfluorinated or optionally substituted by a perfluoroalkyl group; a cycloalkyl group optionally substituted by alkyl or alkoxy; an aryl group optionally substituted by alkyl or alkoxy; an alkenyl group; an alkynyl group; or an arylalkyl group in which the aryl part is optionally substituted by alkyl or alkoxy; or else
- T₁ and T₂ independently represent a monovalent radical of following formula (III):
**-V1-V2** **(III)**
in which:
• V1 is a saturated or unsaturated divalent hydrocarbon group,
• V2 is a monovalent group chosen from the group of the following substituents:
◆ alkoxy, -OR^{a}, with R^{a} corresponding to hydrogen, alkyl or aryl;
◆ silyl, -Si (OR^{b})ₓ(R^{c})₃₋ₓ, with R^{b} corresponding to hydrogen, alkyl, silyl or siloxanyl, R^{c} corresponding to alkyl, aryl and x being an integer between 0 and 3;
◆ amine, alkyl or aryl; or alternatively;
- the substituents T₁, T₂, T₃, T_{3'}, T₄ and T_{4'} can form, in pairs, when they are located on two adjacent ring members in the formulae (II) and (II'), a saturated or unsaturated hydrocarbon chain.
